# EUROPEAN PATENT SPECIFICATION

(11) **EP 0 626 370 B1**
(45) Date of publication and mention of the grant of the patent: **11.02.1998**
(21) Application number: 94108090.5
(22) Date of filing: 25.05.1994
(51) Int. Cl.: C07C 331/28, C07D 265/16

(54) **Process for producing aromatic isothiocyanate derivatives**
Verfahren zur Herstellung von aromatischen Isothiocyanatderivaten
Procédé pour la préparation de dérivés des isothiocyanates aromatiques

(30) Priority: 25.05.1993 JP 122433/93
(43) Date of publication of application: 30.11.1994
(73) Proprietor: SUMITOMO CHEMICAL COMPANY LIMITED, Osaka-shi, Osaka 541 (JP)
(72) Inventor: Matsumoto, Mitsuhiro, Ibaraki-shi, Osaka-fu (JP); Fujita, Kunihiko, Misawa-shi, Aomori-ken (JP)
(74) Representative: VOSSIUS & PARTNER

(56) References cited:
- EP-A- 0 176 101
- EP-A- 0 290 902
- EP-A- 0 304 920
- EP-A- 0 523 244
- GB-A- 985 297
- GB-A- 1 024 913
- GB-A- 2 005 268
- US-A- 5 043 442
- 'HOUBEN-WEYL Methoden der Organischen Chemie, Vol. E4' 1983 , GEORG THIEME VERLAG , STUTTGART, DE * page 853 - page 858 *

## Description

The present invention relates to a process for producing aromatic isothiocyanate derivatives.

Aromatic isothiocyanate derivatives are useful compounds as the intermediates for the production of agricultural chemicals. For the production of an isothiocyanate derivative from an amine derivative, various processes have been known in which, for example, (1) an amine derivative is reacted with thiophosgene (see, e.g., Shin-jikken Kagaku Koza, vol. 14 (III), pp. 1503-1509 (1978), published from Maruzen); (2) an amine derivative such as aniline, bromoaniline or methoxyaniline is reacted with carbon disulfide in the presence of a base using a solvent such as toluene or ether, and then reacted with a chlorinated oxidizing agent (see, e.g., *J. Org. Chem.,* **29**, 3098 (1964)); or (3) an aniline derivative such as 4-chloro-2-fluoro-5-methoxycarbonylmethylthioaniline is reacted with carbon disulfide in the presence of a fused-ring compound such as 1,4-diazabicyclo[2.2.2]octane using a solvent such as toluene or dichloromethane, and then reacted with a chlorinated oxidizing agent (see, e.g., WO 92/13835).

However, the process (1) has a disadvantage that it is necessary to use thiophosgene which is expensive and not readily available. The process (2) has a disadvantage that when 6-amino-7-fluoro-4-(2-propynyl)-2H-1,4-benzoxazin-3(4H)-one or 4-chloro-2-fluoro-5-(2-propynyl)oxyaniline, each of which is an amine derivative in the present invention, is used as the amine derivative, the corresponding isothiocyanate derivative can be obtained only in significantly decreased yield. Further, the process (3) requires the use of a particular fused-ring compound, and it cannot be said that this process has high efficiency from an industrial point of view.

GB-A-985 297 discloses a process for preparing aromatic diisothiocyanates wherein an alkali metal chlorite is used as oxidizing agent. GB-A-1024913 discloses a process for the production of organic isothiocyanates wherein chloramine-T is used as oxidizing agent.

Accordingly, there has been a great demand for developing a process for producing isothiocyanate derivatives in high yield without using thiophosgene, which is expensive and not readily available, or without using any particular fused-ring compound.

This object could be achieved on the basis of the finding that isothiocyanate derivatives can be produced from the corresponding amine derivatives in high yield in the presence of a conventional base in a nitrogen-containing polar solvent.

Thus, the present invention provides a process for producing an aromatic isothiocyanate derivative of the general formula [I]: 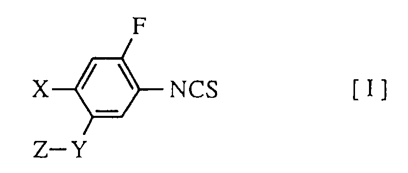 wherein X is chlorine or -O-; when X is chlorine, Y is -O- or -S-, and Z is hydrogen, alkyl, cycloalkyl, alkenyl, alkynyl, alkoxycarbonyl, cycloalkoxycarbonyl, carboxyalkyl, alkoxycarbonylalkyl or cycloalkoxycarbonylalkyl, or when X is -O-, X and Z are taken together to form -O-CH₂-CO-, and Y is -NR- wherein R is hydrogen, alkyl, cycloalkyl, alkenyl or alkynyl, which process comprises reacting an aromatic amine derivative of the general formula [II]: 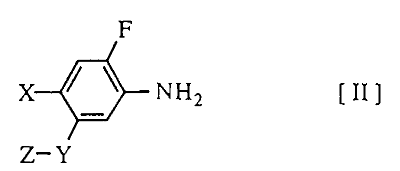 wherein X, Y and Z are each as defined above, with carbon disulfide in the presence of a base in a nitrogen-containing polar solvent, and then with an alkyl chlorocarbonate.

In the production process of the present invention, an aromatic amine derivative of the general formula [II]: 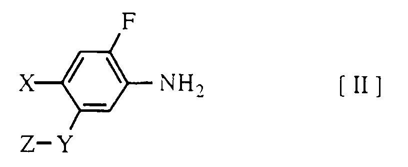 is used as the starting material. In the general formula [II], X is chlorine or -O-. When X is chlorine, Y is -O- or -S-, in which case Z is, for example, hydrogen; C₁-C₆ alkyl such as methyl, ethyl, propyl, iso-propyl, butyl, t-butyl, sec-butyl, pentyl, 2-methylbutyl or hexyl; C₃-C₆ cycloalkyl such as cyclopropyl, cyclopentyl or cyclohexyl; C₃-C₅ alkenyl such as 2-propenyl, 1-buten-3-yl, 2-methyl- 1-propen-3-yl, trans-2-buten-4-yl or cis-2-buten-4-yl; C₃-C₅ alkynyl such as 2-propynyl, 1-butyn-3-yl or 2-butyn-4-yl; (C₁-C₅ alkoxy)carbonyl such as methoxycarbonyl, ethoxycarbonyl, propoxycarbonyl, isopropoxycarbonyl, butoxycarbonyl, sec-butoxycarbonyl, t-butoxycarbonyl or pentyloxycarbonyl; (C₃-C₅ cycloalkoxy)carbonyl such as cyclopropoxycarbonyl or cyclopentyloxycarbonyl; carboxy(C₁-C₆ alkyl) such as carboxymethyl, 2-carboxyethyl or 1-carboxyethyl; (C₁-C₆ alkoxy)carbonyl(C₁-C₆ alkyl) such as methoxycarbonylmethyl, ethoxycarbonylmethyl, propoxycarbonylmethyl, isopropoxycarbonylmethyl, butoxycarbonylmethyl, sec-butoxycarbonylmethyl, t-butoxycarbonylmethyl, pentyloxycarbonylmethyl, hexyloxycarbonylmethyl, 1-methoxycarbonylethyl, 1-ethoxycarbonylethyl, 1-propoxycarbonylethyl, 1-isopropoxycarbonylethyl, 1-butoxycarbonylethyl, 1-sec-butoxycarbonylethyl, 1-t-butoxycarbonylethyl, 1-pentyloxycarbonylethyl, 1-hexyloxycarbonylethyl, 2-methoxycarbonylethyl, 2-ethoxycarbonylethyl, 2-propoxycarbonylethyl, 2-isopropoxycarbonylethyl, 2-butoxycarbonylethyl, 2-sec-butoxycarbonyl ethyl, 2-t-butoxycarbonylethyl, 2-pentyloxycarbonylethyl or 2-hexyloxycarbonylethyl ; or (C₃-C₆ cycloalkoxy)-carbonyl(C₁-C₆ alkyl) such as cyclopropoxycarbonylmethyl, cyclopentyloxycarbonylmethyl, cyclohexyloxycarbonylmethyl, 1-cyclopropoxycarbonylethyl, 1-cyclopentyloxycarbonylethyl, 1-cyclohexyloxycarbonylethyl, 2-cyclopropoxycarbonylethyl, 2-cyclopentyloxycarbonylethyl or 2-cyclohexyloxycarbonylethyl.

When X is -O-, Z and X are taken together to form -O-CH₂-CO-, and Y is -NR-, in which case R is, for example, hydrogen; C₁-C₅ alkyl such as methyl, ethyl, propyl, isopropyl, butyl, t-butyl, sec-butyl, pentyl or 2-methylbutyl; C₃-C₅ cycloalkyl such as cyclopropyl or cyclopentyl; C₃-C₅ alkenyl such as 2-propenyl, 1-buten-3-yl, 2-methyl-1-propen-3-yl, trans-2-buten-4-yl or cis-2-buten-4-yl; or C₃-C₅ alkynyl such as 2-propynyl, 1-butyn-3-yl or 2-butyn-4-yl.

Examples of the aromatic amine derivative [II] include 4-chloro-2-fluoro-5-hydroxyaniline, 4-chloro-2-fluoro-5-methoxyaniline, 4-chloro-2-fluoro-5-ethoxyaniline, 4-chloro-2-fluoro-5-propoxyaniline, 4-chloro-2-fluoro-5-isopropoxyaniline, 4-chloro-2-fluoro-5-cyclopropoxyaniline, 4-chloro-2-fluoro-5-butoxyaniline, 4-chloro-2-fluoro-5-sec-butoxyaniline, 4-chloro-2-fluoro-5-t-butoxyaniline, 4-chloro-2-fluoro-5-pentyloxyaniline, 4-chloro-2-fluoro-5-(2-methylbutyl)oxyaniline, 4-chloro-2-fluoro-5-cyclopentyloxyaniline, 4-chloro-2-fluoro-5-(2-propenyl)oxyaniline, 4-chloro-2-fluoro-5-(1-buten-3-yl)oxyaniline, 4-chloro-2-fluoro-5-(2-methyl-1-propen-3-yl)oxyaniline, 4-chloro-2-fluoro-5-(trans-2-buten-4-yl)oxyaniline, 4-chloro-2-fluoro-5-(cis-2-buten-4-yl)oxyaniline, 4-chloro-2-fluoro-5-(2-propynyl)oxyaniline, 4-chloro-2-fluoro-5-(1-butyn-3-yl)oxyaniline, 4-chloro-2-fluoro-5-(2-butyn-4-yl)oxyaniline, 4-chloro-2-fluoro-5-methoxycarbonyloxyaniline, 4-chloro-2-fluoro-5-ethoxycarbonyloxyaniline, 4-chloro-2-fluoro-5-propoxycarbonyloxyaniline, 4-chloro-2-fluoro-5-isopropoxycarbonyloxyaniline, 4-chloro-2-fluoro-5-cyclopropoxycarbonyloxyaniline, 4-chloro-2-fluoro-5-butoxycarbonyloxyaniline, 4-chloro-2-fluoro-5-sec-butoxycarbonyloxyaniline, 4-chloro-2-fluoro-5-t-butoxycarbonyloxyaniline, 4-chloro-2-fluoro-5-pentyloxycarbonyloxyaniline, 4-chloro-2-fluoro-5-cyclopentyloxycarbonyloxyaniline, 4-chloro-2-fluoro-5-carboxymethoxyaniline, 4-chloro-2-fluoro-5-methoxycarbonylmethoxyaniline, 4-chloro-2-fluoro-5-ethoxycarbonylmethoxyaniline, 4-chloro-2-fluoro-5-propoxycarbonylmethoxyaniline, 4-chloro-2-fluoro-5-isopropoxycarbonylmethoxyaniline, 4-chloro-2-fluoro-5-cyclopropoxycarbonylmethoxyaniline, 4-chloro-2-fluoro-5-butoxycarbonylmethoxyaniline, 4-chloro-2-fluoro-5-secbutoxycarbonylmethoxyaniline, 4-chloro-2-fluoro-5-t-butoxycarbonylmethoxyaniline, 4-chloro-2-fluoro-5-pentyloxycarbonylmethoxyaniline, 4-chloro-2-fluoro-5-cyclopentyloxycarbonylmethoxyaniline, 4-chloro-2-fluoro-5(1'-carboxy)ethoxyaniline, 4-chloro-2-fluoro-5(1'-methoxycarbonyl)ethoxyaniline, 4-chloro-2-fluoro-5(1'-ethoxycarbonyl)ethoxyaniline, 4-chloro-2-fluoro-5(1'-isopropoxycarbonyl)ethoxyaniline, 4-chloro-2-fluoro-5(1'-cyclopropoxycarbonyl)ethoxyaniline, 4-chloro-2-fluoro-5(1'-butoxycarbonyl)ethoxyaniline, 4-chloro-2-fluoro-5-(1'-t-butoxycarbonyl)ethoxyaniline, 4-chloro-2-fluoro-5(1'-pentyloxycarbonyl)ethoxyaniline, 4-chloro-2-fluoro-5(1'-cyclopentyloxycarbonyl)ethoxyaniline, 4-chloro-2-fluoro-5-mercaptoaniline, 4-chloro-2-fluoro-5-methylthioaniline, 4-chloro-2-fluoro-5-ethylthioaniline, 4-chloro-2-fluoro-5-propylthioaniline, 4-chloro-2-fluoro-5-isopropylthioaniline, 4-chloro-2-fluoro-5-cyclopropylthioaniline, 4-chloro-2-fluoro-5-buthylthioaniline, 4-chloro-2-fluoro-5-sec-butylthioaniline, 4-chloro-2-fluoro-5-t-butylthioaniline,4-chloro-2-fluoro-5-pentylthioaniline, 4-chloro-2-fluoro-5-(2-methylbutyl)thioaniline,4-chloro-2-fluoro-5-cyclopentylthioaniline, 4-chloro-2-fluoro-5-(2-propenyl)thioaniline, 4-chloro-2-fluoro-5-(1-buten-3-yl)thioaniline, 4-chloro-2-fluoro-5-(2-methyl-1-propen-3-yl)thioaniline, 4-chloro-2-fluoro-5-(trans-2-buten-4-yl)thioaniline, 4-chloro-2-fluoro-5-(cis-2-buten-4-yl)thioaniline, 4-chloro-2-fluoro-5-(2-propynyl)thioaniline, 4-chloro-2-fluoro-5-(1-butyn-3-yl)thioaniline, 4-chloro-2-fluoro-5-(2-butyn-4-yl)thioaniline, 4-chloro-2-fluoro-5-methoxycarbonylthioaniline, 4-chloro-2-fluoro-5-ethoxycarbonylthioaniline, 4-chloro-2-fluoro-5-propoxycarbonylthioaniline, 4-chloro-2-fluoro-5-isopropoxycarbonylthioaniline, 4-chloro-2-fluoro-5-cyclopropoxycarbonylthioaniline, 4-chloro-2-fluoro-5-butoxycarbonylthioaniline, 4-chloro-2-fluoro-5-sec-butoxycarbonylthioaniline, 4-chloro-2-fluoro-5-t-butoxycarbonylthioaniline, 4-chloro-2-fluoro-5-pentyloxycarbonylthioaniline, 4-chloro-2-fluoro-5-cyclopentyloxycarbonylthioaniline, 4-chloro-2-fluoro-5-carboxymethylthioaniline, 4-chloro-2-fluoro-5-methoxycarbonylmethylthioaniline, 4-chloro-2-fluoro-5-ethoxycarbonylmethylthioaniline, 4-chloro-2-fluoro-5-propoxycarbonylmethylthioaniline, 4-chloro-2-fluoro-5-isopropoxycarbonylmethylthioaniline, 4-chloro-2-fluoro-5-cyclopropoxycarbonylmethylthioaniline, 4-chloro-2-fluoro-5-butoxycarbonylmethylthioaniline, 4-chloro-2-fluoro-5-sec-butoxycarbonylmethylthioaniline, 4-chloro-2-fluoro-5-t-butoxycarbonylmethylthioaniline, 4-chloro-2-fluoro-5-pentyloxycarbonylmethylthioaniline, 4-chloro-2-fluoro-5-cyclopentyloxycarbonylmethylthioaniline, 4-chloro-2-fluoro-5(1'-carboxy)ethylthioaniline, 4-chloro-2-fluoro-5(1'-methoxycarbonyl)ethylthioaniline, 4-chloro-2-fluoro-5(1'-ethoxycarbonyl)ethylthioaniline, 4-chloro-2-fluoro-5(1'-isopropoxycarbonyl)ethylthioaniline, 4-chloro-2-fluoro-5(1'-cyclopropoxycarbonyl)ethylthioaniline, 4-chloro-2-fluoro-5(1'-butoxycarbonyl)ethylthioaniline, 4-chloro-2-fluoro-5(1'-t-butoxycarbonyl)ethylthioaniline, 4-chloro-2-fluoro-5(1'-pentyloxycarbonyl)ethylthioaniline, 4-chloro-2-fluoro-5(1'-cyclopentyloxycarbonyl)ethylthioaniline, 6-amino-7-fluoro-2H-1,4-benzoxazin-3(4H)-one, 6-amino-7-fluoro-4-methyl-2H-1,4-benzoxazin-3(4H)-one, 6-amino-7-fluoro-4-ethyl-2H- 1,4-benzoxazin-3(4H)-one, 6-amino-7-fluoro-4-propyl-2H-1,4-benzoxazin-3(4H)-one, 6-amino-7-fluoro-4-isopropyl-2H-1,4-benzoxazin-3(4H)-one, 6-amino-7-fluoro-4-cyclopropyl-2H-1,4-benzoxazin-3(4H)-one, 6-amino-7-fluoro-4-butyl-2H-1,4-benzoxazin-3(4H)-one, 6-amino-7-fluoro-4-(t-butyl)-2H-1,4-benzoxazin-3(4H)-one, 6-amino-7-fluoro-4-(sec-butyl)-2H-1,4-benzoxazin-3(4H)-one, 6-amino-7-fluoro-4-pentyl-2H-1,4-benzoxazin-3(4H)-one, 6-amino-7-fluoro-4-(2-methylbutyl)-2H-1,4-benzoxazin-3(4H)-one, 6-amino-7-fluoro-4-cyclopentyl-2H-1,4-benzoxazin-3(4H)-one, 6-amino-7-fluoro-4-(2-propenyl)-2H-1,4-benzoxazin-3(4H)-one, 6-amino-7-fluoro-4-(1-buten-3-yl)-2H-1,4-benzoxazin-3(4H)-one, 6-amino-7-fluoro-4-(2-methyl-1-propen-3-yl)-2H-1,4-benzoxazin-3(4H)-one, 6-amino-7-fluoro-4-(trans-2-buten-4-yl)-2H-1,4-benzoxazin-3(4H)-one, 6-amino-7-fluoro-4-(cis-2-buten-4-yl)-2H-1,4-benzoxazin-3(4H)-one, 6-amino-7-fluoro-4-(2-propynyl)-2H-1,4-benzoxazin-3(4H)-one, 6-amino-7-fluoro-4-(1-butyn-3-yl)-2H-1,4-benzoxazin-3(4H)-one and 6-amino-7-fluoro-4-(2-butyn-4-yl)-2H-1,4-benzoxazin-3(4H)-one.

The process of the present invention is characterized by the use of a nitrogen-containing polar solvent in the production of an aromatic isothiocyanate derivative [ I ]. The aromatic isothiocyanate derivative [I] can be produced by the reaction of an aromatic amine derivative [II] with carbon disulfide in the presence of a base in a nitrogen-containing polar solvent, and then with an alkyl chlorocarbonate as chlorinated oxidizing agent.

Examples of the nitrogen-containing polar solvent include heterocyclic compounds such as 1-methylimidazole, pyridine, 5-ethyl-2-methylpyridine, 2-picoline, 3-picoline and 4-picoline; and amido compounds such as N,N-dimethylformamide and 1-methyl-2-pyrrolidone. Taking into consideration the yield of an aromatic isothiocyanate derivative [I] and from an economical point of view, the use of pyridine, 4-picoline or N,N-dimethylformamide is preferred.

The amount of nitrogen-containing polar solvent to be used is usually 2 to 30 times the weight of aromatic amine derivative [II]. The nitrogen-containing polar solvent may be used alone or in combination with any other organic solvent or water.

Examples of the base include inorganic bases such as sodium hydroxide, potassium hydroxide, sodium carbonate, potassium carbonate, sodium acetate and potassium acetate; and organic bases such as triethylamine, trimethylamine, 1-methylpiperidine, 1-methylpyrrolidine, 4-methylmorpholine and 4-dimethylaminopyridine. Particularly preferred are inorganic bases such as sodium carbonate and potassium carbonate; and organic bases such as triethylamine, trimethylamine, 1-methylpiperidine and 4-dimethylaminopyridine. These bases may be used alone or in combination with each other.

When an inorganic base is used, the addition of a phase transfer catalyst such as tetrabutylammonium bromide is preferred.

The amount of base to be used is usually about 1 to 10 times the mol of aromatic amine derivative [II].

The amount of carbon disulfide to be used is usually about 1 to 10 times the mol of aromatic amine derivative [II].

The order of adding an aromatic amine derivative [II], a base and carbon disulfide is not particularly limited; however, the aromatic amine derivative [II] is usually added to a solvent, to which carbon disulfide and the base are then added to effect the reaction. The reaction is usually carried out at a temperature of 0° to 40°C for about 2 to 20 hours.

The subsequent reaction is effected with a chlorinated oxidizing agent, i.e. an alkyl chlorocarbonate. Examples of such alkyl chlorocarbonates are methyl chlorocarbonate, ethyl chlorocarbonate, propyl chlorocarbonate and isopropyl chlorocarbonate.

The amount of chlorinated oxidizing agent to be used is usually about 1 to 10 times the mol of aromatic amine derivative [II]. The reaction is usually carried out at a temperature of 0° to 30°C for about 5 minutes to 20 hours.

After completion of the reaction, the desired isothiocyanate derivative [I] can be obtained by ordinary post-treatment. For example, the reaction mixture is extracted with an appropriate organic solvent and the extract is concentrated by distillation of the organic solvent. The isothiocyanate derivative [I] thus obtained may be purified by any conventional purification technique.

Examples of the isothiocyanate derivative [I] to be produced are 4-chloro-2-fluoro-5-hydroxyphenylisothiocyanate,4-chloro-2-fluoro-5-methoxyphenylisothiocyanate, 4-chloro-2-fluoro-5-ethoxyphenylisothiocyanate, 4-chloro-2-fluoro-5-propoxyphenylisothiocyanate, 4-chloro-2-fluoro-5-isopropoxyphenylisothiocyanate, 4-chloro-2-fluoro-5-cyclopropoxyphenylisothiocyanate, 4-chloro-2-fluoro-5-butoxyphenyl isothiocyanate, 4-chloro-2-fluoro-5-sec-butoxyphenylisothiocyanate, 4-chloro-2-fluoro-5-t-butoxyphenylisothiocyanate. 4-chloro-2-fluoro-5-pentyloxyphenylisothiocyanate, 4-chloro-2-fluoro-5-(2-methylbutyl)oxyphenylisothiocyanate, 4-chloro-2-fluoro-5-cyclopentyloxyphenylisothiocyanate, 4-chloro-2-fluoro-5-(2-propenyl)oxyphenylisothiocyanate, 4-chloro-2-fluoro-5-(1-buten-3-yl)oxyphenylisothiocyanate, 4-chloro-2-fluoro-5-(2-methyl-1-propen-3-yl )oxyphenylisothiocyanate, 4-chloro-2-fluoro-5-(trans-2-buten-4-yl)oxyphenylisothiocyanate, 4-chloro-2-fluoro-5-(cis-2-buten-4-yl)oxyphenylisothiocyanate, 4-chloro-2-fluoro-5-(2-propynyl)oxyphenylisothiocyanate, 4-chloro-2-fluoro-5-(1-butyn-3-yl)oxyphenylisothiocyanate, 4-chloro-2-fluoro-5-(2-butyn-4-yl)oxyphenylisothiocyanate, 4-chloro-2-fluoro-5-methoxycarbonyloxyphenylisothiocyanate, 4-chloro-2-fluoro-5-ethoxycarbonyloxyphenylisothiocyanate, 4-chloro-2-fluoro-5-propoxycarbonyloxyphenylisothiocyanate, 4-chloro-2-fluoro-5-isopropoxycarbonyloxyphenylisothiocyanate, 4-chloro-2-fluoro-5-cyclopropoxycarbonyloxyphenylisothiocyanate, 4-chloro-2-fluoro-5-butoxycarbonyloxyphenylisothiocyanate, 4-chloro-2-fluoro-5-sec-butoxycarbonyloxyphenylisothiocyanate, 4-chloro-2-fluoro-5-t-butoxycarbonyloxyphenylisothiocyanate, 4-chloro-2-fluoro-5-pentyloxycarbonyloxyphenylisothiocyanate, 4-chloro-2-fluoro-5-cyclopentyloxycarbonyloxyphenylisothiocyanate, 4-chloro-2-fluoro-5-carboxymethoxyphenylisothiocyanate, 4-chloro-2-fluoro-5-methoxycarbonylmethoxyphenylisothiocyanate, 4-chloro-2-fluoro-5-ethoxycarbonylmethoxyphenylisothiocyanate, 4-chloro-2-fluoro-5-propoxycarbonylmethoxyphenylisothiocyanate, 4-chloro-2-fluoro-5-isopropoxycarbonylmethoxyphenylisothiocyanate, 4-chloro-2-fluoro-5-cyclopropoxycarbonylmethoxyphenylisothiocyanate, 4-chloro-2-fluoro-5-butoxycarbonylmethoxyphenylisothiocyanate, 4-chloro-2-fluoro-5-sec-butoxycarbonylmethoxyphenylisothiocyanate, 4-chloro-2-fluoro-5-t-butoxycarbonylmethoxyphenylisothiocyanate, 4-chloro-2-fluoro-5-pentyloxycarbonylmethoxyphenylisothiocyanate, 4-chloro-2-fluoro-5-cyclopentyloxycarbonylmethoxyphenylisothiocyanate, 4-chloro-2-fluoro-5(1'-carboxy)ethoxyphenylisothiocyanate, 4-chloro-2-fluoro-5(1'-methoxycarbonyl )ethoxyphenylisothiocyanate, 4-chloro-2-fluoro-5(1'-ethoxycarbonyl)ethoxyphenylisothiocyanate, 4-chloro-2-fluoro-5(1'-isopropoxycarbonyl)ethoxyphenylisothiocyanate, 4-chloro-2-fluoro-5(1'-cyclopropoxycarbonyl)ethoxyphenylisothiocyanate, 4-chloro-2-fluoro-5(1'-butoxycarbonyl)ethoxyphenylisothiocyanate, 4-chloro-2-fluoro-5(1'-t-butoxycarbonyl)ethoxyphenylisothiocyanate, 4-chloro-2-fluoro-5(1'-pentyloxycarbonyl)ethoxyphenylisothiocyanate, 4-chloro-2-fluoro-5(1'-cyclopentyloxycarbonyl)ethoxyphenylisothiocyanate, 4-chloro-2-fluoro-5-mercaptophenylisothiocyanate, 4-chloro-2-fluoro-5-methylthiophenylisothiocyanate, 4-chloro-2-fluoro-5-ethylthiophenylisothiocyanate, 4-chloro-2-fluoro-5-propylthiophenylisothiocyanate, 4-chloro-2-fluoro-5-isopropylthiophenylisothiocyanate, 4-chloro-2-fluoro-5-cyclopropylthiophenylisothiocyanate, 4-chloro-2-fluoro-5-butylthiophenylisothiocyanate, 4-chloro-2-fluoro-5-sec-butylthiophenylisothiocyanate, 4-chloro-2-fluoro-5-t-butylthiophenylisothiocyanate, 4-chloro-2-fluoro-5-pentylthiophenylisothiocyanate, 4-chloro-2-fluoro-5-(2-methylbutyl)thiophenylisothiocyanate, 4-chloro-2-fluoro-5-cyclopentylthiophenylisothiocyanate, 4-chloro-2-fluoro-5-(2-propenyl)thiophenylisothiocyanate, 4-chloro-2-fluoro-5-(1-buten-3-yl)thiophenylisothiocyanate, 4-chloro-2-fluoro-5-(2-methyl-1-propen-3-yl)thiophenylisothiocyanate, 4-chloro-2-fluoro-5-(trans-2-buten-4-yl)thiophenylisothiocyanate, 4-chloro-2-fluoro-5-(cis-2-buten-4-yl)thiophenylisothiocyanate, 4-chloro-2-fluoro-5-(2-propynyl)thiophenylisothiocyanate, 4-chloro-2-fluoro-5-(1-butyn-3-yl)thiophenylisothiocyanate, 4-chloro-2-fluoro-5-(2-butyn-4-yl)thiophenylisothiocyanate, 4-chloro-2-fluoro-5-methoxycarbonylthiophenylisothiocyanate, 4-chloro-2-fluoro-5-ethoxycarbonylthiophenylisothiocyanate, 4-chloro-2-fluoro-5-propoxycarbonylthiophenylisothiocyanate, 4-chloro-2-fluoro-5-isopropoxycarbonylthiophenylisothiocyanate, 4-chloro-2-fluoro-5-cyclopropoxycarbonylthiophenylisothiocyanate, 4-chloro-2-fluoro-5-butoxycarbonylthiophenylisothiocyanate, 4-chloro-2-fluoro-5-sec-butoxycarbonylthiophenylisothiocyanate, 4-chloro-2-fluoro-5-t-butoxycarbonylthiophenylisothiocyanate, 4-chloro-2-fluoro-5-pentyloxycarbonylthiophenylisothiocyanate, 4-chloro-2-fluoro-5-cyclopentyloxycarbonylthiophenylisothiocyanate, 4-chloro-2-fluoro-5-carboxymethylthiophenylisothiocyanate, 4-chloro-2-fluoro-5-methoxycarbonylmethylthiophenylisothiocyanate, 4-chloro-2-fluoro-5-ethoxycarbonylmethylthiophenylisothiocyanate, 4-chloro-2-fluoro-5-propoxycarbonylmethylthiophenylisothiocyanate, 4-chloro-2-fluoro-5-isopropoxycarbonylmethylthiophenylisothiocyanate, 4-chloro-2-fluoro-5-cyclopropoxycarbonylmethylthiophenylisothiocyanate, 4-chloro-2-fluoro-5-butoxycarbonylmethylthiophenylisothiocyanate, 4-chloro-2-fluoro-5-sec-butoxycarbonylmethylthiophenylisothiocyanate, 4-chloro-2-fluoro-5-t-butoxycarbonylmethylthiophenylisothiocyanate, 4-chloro-2-fluoro-5-pentyloxycarbonylmethylthiophenylisothiocyanate, 4-chloro-2-fluoro-5-cyclopentyloxycarbonylmethylthiophenylisothiocyanate, 4-chloro-2-fluoro-5(1'-carboxy)ethylthiophenylisothiocyanate, 4-chloro-2-fluoro-5(1'-methoxycarbonyl)ethylthiophenylisothiocyanate, 4-chloro-2-fluoro-5(1'-ethoxycarbonyl)ethylthiophenylisothiocyanate, 4-chloro-2-fluoro-5(1'-isopropoxycarbonyl)ethylthiophenylisothiocyanate, 4-chloro-2-fluoro-5(1'-cyclopropoxycarbonyl)ethylthiophenylisothiocyanate, 4-chloro-2-fluoro-5(1'-butoxycarbonyl)ethylthiophenylisothiocyanate, 4-chloro-2-fluoro-5(1'-t-butoxycarbonyl)ethylthiophenylisothiocyanate, 4-chloro-2-fluoro-5(1'-pentyloxycarbonyl)ethylthiophenylisothiocyanate, 4-chloro-2-fluoro-5(1'-cyclopentyloxycarbonyl)ethylthiophenylisothiocyanate, 7-fluoro-6-isothiocyanato-2H-1,4-benzoxazin-3(4H)-one, 7-fluoro-6-isothiocyanato-4-methyl-2H-1,4-benzoxazin-3(4H)-one, 7-fluoro-6-isothiocyanato-4-ethyl-2H-1,4-benzoxazin-3(4H)-one,7-fluoro-6-isothiocyanato-4-propyl-2H-1,4-benzoxazin-3(4H)-one, 7-fluoro-6-isothiocyanato-4-isopropyl-2H-1,4-benzoxazin-3(4H)-one, 7-fluoro-6-isothiocyanato-4-cyclopropyl-2H-1,4-benzoxazin-3(4H)-one, 7-fluoro-6-isothiocyanato-4-butyl-2H-1,4-benzoxazin-3(4H)-one, 7-fluoro-6-isothiocyanato-4-(t-butyl)-2H-1,4-benzoxazin-3(4H)-one, 7-fluoro-6-isothiocyanato-4-(sec-butyl)-2H-1,4-benzoxazin-3(4H)-one, 7-fluoro-6-isothiocyanato-4-pentyl-2H-1,4-benzoxazin-3(4H)-one, 7-fluoro-6-isothiocyanato-4-(2-methylbutyl)-2H-1,4-benzoxazin-3(4H)-one, 7-fluoro-6-isothiocyanato-4-cyclopentyl-2H-1,4-benzoxazin-3(4H)-one, 7-fluoro-6-isothiocyanato-4-(2-propenyl)-2H-1,4-benzoxazin-3(4H)-one, 7-fluoro-6-isothiocyanato-4-(1-buten-3-yl)-2H-1,4-benzoxazin-3(4H)-one, 7-fluoro-6-isothiocyanato-4-(2-methyl-1-propen-3-yl)-2H-1,4-benzoxazin-3(4H)-one, 7-fluoro-6-isothiocyanato-4-(trans-2-buten-4-yl)-2H-1,4-benzoxazin-3(4H)-one, 7-fluoro-6-isothiocyanato-4-(cis-2-buten-4-yl)-2H-1,4-benzoxazin-3(4H)-one, 7-fluoro-6-isothiocyanato-4-(2-propynyl)-2H-1,4-benzoxazin-3(4H)-one, 7-fluoro-6-isothiocyanato-4-(1-butyn-3-yl)-2H-1,4-benzoxazin-3(4H)-one and 7-fluoro-6-isothiocyanato-4-(2-butyn-4-yl)-2H-1,4-benzoxazin-3(4H)-one.

The present invention makes it possible to produce an aromatic isothiocyanate derivative [I] from the corresponding aromatic amine derivative [II] in the present of a conventional base in a nitrogen-containing polar solvent in high yield.

The present invention will be further illustrated by way of the following examples, which are not to be construed to limit the scope thereof.

The yield of aromatic isothiocyanate derivative [I] was based on the amount of aromatic amine derivative [II].

### Example I

A 20-ml two-necked flask was charged with 0.50 g (2.5 mmol) of 4-chloro-2-fluoro-5-(2-propynyl)oxyaniline, to which 4 g of pyridine was added and dissolved. Then, 0.57 g (7.5 mmol) of carbon disulfide and 0.76 g (7.5 mmol) of triethylamine were added thereto with stirring at 25°C, and the stirring was continued at the same temperature for 16 hours. The reaction mass was cooled to 0°C, to which 0.47 g (5 mmol) of methyl chlorocarbonate was added dropwise. After further stirring at 0°C for I hour, 20 g of water and 30 g of ethyl acetate were added thereto, followed by extraction. To the aqueous layer, 20 g of ethyl acetate was further added, and the extraction was carried out twice. All the ethyl acetate layers were combined together, to which anhydrous sodium sulfate was added for drying, and the solvent was distilled off under reduced pressure, which afforded 4-chloro-2-fluoro-5-(2-propynyl)oxyphenylisothiocyanate

The analysis by gas chromatography revealed that the yield of 4-chloro-2-fluoro-5-(2-propynyl)oxyphenylisothiocyanate was 71%.

### Example 2

4-Chloro-2-fluoro-5-pentyloxycarbonylmethoxyphenylisothiocyanate was produced in the same manner as described in Example 1, except that 0.72 g (2.5 mmol) of 4-chloro-2-fluoro-5-pentyloxycarbonylmethoxyaniline was used in place of 0.50 g (2.5 mmol) of 4-chloro-2-fluoro-5-(2-propynyl)oxyaniline.

The analysis by gas chromatography revealed that the yield of 4-chloro-2-fluoro-5-pentyloxycarbonylmethoxyphenylisothiocyanate was 76%.

### Example 3

4-Chloro-2-fluoro-5-cyclopentyloxycarbonylmethylthiophenylisothiocyanate was produced in the same manner as described in Example 1, except that 0.76 g (2.5 mmol) of 4-chloro-2-fluoro-5-cyclopentyloxycarbonylmethylthioaniline was used in place of 0.50 g (2.5 mmol) of 4-chloro-2-fluoro-5-(2-propynyl)oxyaniline.

The analysis by gas chromatography revealed that the yield of 4-chloro-2-fluoro-5-cyclopentyloxycarbonylmethylthiophenylisothiocyanate was 70%.

### Comparative Example 1

A 20-ml two-necked flask was charged with 0.40 g (2.5 mmol) of 4-chloro-2-fluoro-5-hydroxyaniline, to which 4 g of pyridine was added and dissolved. Then, 0.57 g (7.5 mmol) of carbon disulfide and 0.76 g (7.5 mmol) of triethylamine were added thereto with stirring at 25°C, and the stirring was continued at the same temperature for 16 hours. The reaction mass was cooled to 0°C, to which 7.4 g (10 mmol) of 10% aqueous sodium hypochlorite solution was added dropwise over about 5 minutes with maintaining the same temperature. After further stirring at 0°C for 30 minutes, 20 g of 5% aqueous sodium sulfite solution and 30 g of dichloromethane were added thereto, followed by extraction. To the aqueous layer, 20 g of dichloromethane was further added, and the extraction was carried out twice. All the dichloromethane layers were combined together, to which anhydrous sodium sulfate was added for drying, and the solvent was distilled off under reduced pressure, which afforded 4-chloro-2-fluoro-5-hydroxyphenylisothiocyanate.

The analysis by gas chromatography revealed that the yield of 4-chloro-2-fluoro-5-hydroxyphenylisothiocyanate was 48%.

### Comparative Example 2

4-Chloro-2-fluoro-5-(2-propynyl)oxyphenylisothiocyanate was produced in the same manner as described in Example 1, except that 4 g of toluene and 100 g of ethyl acetate were used in place of 4 g of pyridine and 30 g of ethyl acetate, respectively.

The analysis by gas chromatography revealed that the yield of 4-chloro-2-fluoro-5-(2-propynyl)oxyphenylisothiocyanate was 5%.

### Comparative Example 3

4-Chloro-2-fluoro-5-pentyloxycarbonylmethoxyphenylisothiocyanate was produced in the same manner as described in Example 2, except that 4 g of toluene and 100 g of ethyl acetate were used in place of 4 g of pyridine and 30 g of ethyl acetate, respectively.

The analysis by gas chromatography revealed that the yield of 4-chloro-2-fluoro-5-pentyloxycarbonylmethoxyphenylisothiocyanate was 7%.

### Example 4

A 20-ml two-necked flask was charged with 0.55 g (2.5 mmol) of 6-amino-7-fluoro-4-(2-propynyl)-2H-1,4-benzoxazin-3(4H)-one, to which 3 g of pyridine was added and dissolved. After cooling to 0 °C, 0.57 g (7.5 mmol) of carbon disulfide and 0.76 g (7.5 mmol) triethylamine were added thereto, and the stirring was continued at the same temperature for 3.5 hours. Then, 0.47 g (5 mmol) of methyl chlorocarbonate was added dropwise with maintaining the same temperature. After further stirring at 0°C for 30 minutes, 20 g of water and 30 g of dichoromethane were added thereto, followed by extraction. To the aqueous layer, 20 g of dichloromethane was further added, and the extraction was carried out twice. All the dichloromethane layers were combined together, to which anhydrous sodium sulfate was added for drying, and dichloromethane was removed by evaporation, which afforded 7-fluoro-6-isothiocyanato-4-(2-propynyl)-2H-1,4-benzoxazin-3(4H)-one.

The analysis by gas chromatography revealed that the yield of 7-fluoro-6-isothiocyanato-4-(2-propynyl)-2H-1,4-benzoxazin-3(4H)-one was 93%.

### Example 5

7-Fluoro-6-isothiocyanato-4-(2-propynyl)-2H-1,4-benzoxazin-3(4H)-one was produced in the same manner as described in Example 4, except that 4.5 g of pyridine, 0.95 g (12.5 mmol) of carbon disulfide and 0.74 g (7.5 mmol) of N-methylpiperidine were used in place of 3 g of pyridine, 0.57 g (7.5 mmol) of carbon disulfide and 0.76 g (7.5 mmol) of triethylamine, respectively, and after the addition of N-methylpiperidine, the mixture was stirred at 0°C for 7 hours.

The analysis by gas chromatography revealed that yield of 7-fluoro-6-isothiocyanato-4-(2-propynyl)-2H-1,4-benzoxazin-3(4H)-one was 93%.

### Example 6

A 20-ml two-necked flask was charged with 0.55 g (2.5 mmol) of 6-amino-7-fluoro-4-(2-propynyl)-2H-1,4-benzoxazin-3(4H)-one, to which 0.92 g (7.5 mmol) of 4-dimethylaminopyridine and 4.5 g of N,N-dimethylformamide were added, and the mixture was stirred at 20°C for 5 minutes. After cooling to 0 °C, 0.95 g (12.5 mmol) of carbon disulfide was added thereto, and the stirring was continued at the same temperature for 3 hours, after which the temperature was raised to 20 °C and the stirring was further continued for 10 hours. After cooling to 0 °C, 0.47 g (5 mmol) of methyl chlorocarbonate was added dropwise, and the mixture was treated thereafter in the same manner as described in Example 4, which afforded 7-fluoro-6-isothiocyanato-4-(2-propynyl)-2H-1,4-benzoxazin-3(4H)-one.

The analysis by gas chromatography revealed that the yield of 7-fluoro-6-isothiocyanato-4-(2-propynyl)-2H-1,4-benzoxazin-3(4H)-one was 95%.

### Example 7

7-Fluoro-6-isothiocyanato-4-(2-propynyl)-2H-1,4-benzoxazin-3(4H)-one was produced in the same manner as described in Example 6, except that 5.5 g of 4-picoline was used in place of 4.5 g of N,N-dimethylformamide.

The analysis by gas chromatography revealed that the yield of 7-fluoro-6-isothiocyanato-4-(2-propynyl)-2H-1,4-benzoxazin-3(4H)-one was 93%.

### Example 8

A 20-ml two-necked flask was charged with 0.55 g (2.5 mmol) of 6-amino-7-fluoro-4-(2-propynyl)-2H-1,4-benzoxazin-3(4H)-one, 1.04 g (7.5 mmol) of potassium carbonate, 0.08 g (0.25 mmol) of tetrabutylammonium bromide and 6 g of pyridine. After cooling to 0 °C, 0.95 g (12.5 mmol) of carbon disulfide was added thereto, and the mixture was stirred at 0 °C for 11 hours. Then, 0.47 g (5 mmol) of methyl chlorocarbonate was added dropwise with maintaining the same temperature, and the mixture was treated thereafter in the same manner as described in Example 4, which afforded 7-fluoro-6-isothiocyanato-4-(2-propynyl)-2H-1,4-benzoxazin-3(4H)-one.

The analysis by gas chromatography revealed that the yield of 7-fluoro-6-isothiocyanato-4-(2-propynyl)-2H-1,4-benzoxazin-3(4H)-one was 75%.

### Comparative Example 4

7-Fluoro-6-isothiocyanato-4-(2-propynyl)-2H-1,4-benzoxazin-3(4H)-one was produced in the same manner as described in Example 4, except that 7.3 g (12.5 mmol) of 12.7 % aqueous sodium hypochlorite solution was used in place of 0.47 g (5 mmol) of methyl chlorocarbonate.

The analysis by gas chromatography revealed that the yield of 7-fluoro-6-isothiocyanato-4-(2-propynyl)-2H-1,4-benzoxazin-3(4H)-one was 84%.

### Comparative Example 5

7-Fluoro-6-isothiocyanato-4-(2-propynyl)-2H-1,4-benzoxazin-3(4H)-one was produced in the same manner as described in Example 4, except that 6 g of tetrahydrofuran was used in place of 3 g pyridine, and the reaction of 6-amino-7-fluoro-4-(2-propynyl)-2H-1,4-benzoxazin-3(4H)-one and carbon disulfide was continued for 8 hours.

The analysis by gas chromatography revealed that the yield of 7-fluoro-6-isothiocyanato-4-(2-propynyl)-2H-1,4-benzoxazin-3(4H)-one was 20%.

### Example 9

A 20-ml two-necked flask was charged with 0.49 g (2.5 mmol) of 6-amino-7-fluoro-4-methyl-2H- 1,4-benzoxazin-3(4H)-one, to which 4.5 g of pyridine was added and suspended. Then, 0.57 g (7.5 mmol) of carbon disulfide and 0.76 g (7.5 mmol) of triethylamine were added thereto at room temperature. The mixture was cooled to 0 °C and stirred at the same temperature for 5 hours. The mixture was treated thereafter in the same manner as described in Example 4, which afforded 7-fluoro-6-isothiocyanato-4-methyl-2H-1,4-benzoxazin-3(4H)-one.

The analysis by gas chromatography revealed that the yield of 7-fluoro-6-isothiocyanato-4-methyl-2H-1,4-benzoxazin-3(4H)-one was 91%.

### Comparative Example 6

7-Fluoro-6-isothiocyanato-4-methyl-2H- 1,4-benzoxazin-3(4H)-one was produced in the same manner as described in Example 9, except that 6 g of tetrahydrofuran was used in place of 4.5 g of pyridine.

The analysis by gas chromatography revealed that the yield of 7-fluoro-6-isothiocyanato-4-methyl-2H-1,4-benzoxazin-3(4H)-one was 3%.

## Claims

1. A process for producing an aromatic isothiocyanate derivative of the general formula [I] : 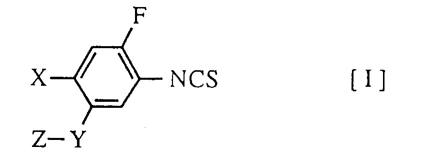 wherein X is chlorine or -O-; when X is chlorine, Y is -O- or -S-, and Z is hydrogen, alkyl, cycloalkyl, alkenyl, alkynyl, alkoxycarbonyl, cycloalkoxycarbonyl, carboxyalkyl, alkoxycarbonylalkyl or cycloalkoxycarbonylalkyl, or when X is -O-, X and Z are taken together to form -O-CH₂-CO-, and Y is -NR- wherein R is hydrogen, alkyl, cycloalkyl, alkenyl or alkynyl, which process comprises reacting an aromatic amine derivative of the general formula [II]: 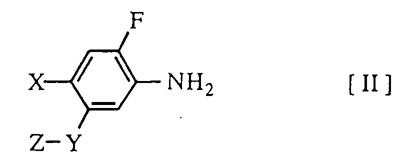 wherein X, Y and Z are each as defined above, with carbon disulfide in the presence of a base in a nitrogen-containing polar solvent, and then with an alkyl chlorocarbonate.

2. A process for producing an aromatic isothiocyanate derivative according to claim 1, wherein X is chlorine, Y is -O- and Z is hydrogen, alkynyl, alkoxycarbonylalkyl or cycloalkoxycarbonylalkyl.

3. A process for producing an aromatic isothiocyanate derivative according to claim 1, wherein X is chlorine, Y is -S- and Z is alkoxycarbonylalkyl or cycloalkoxycarbonylalkyl.

4. A process for producing an aromatic isothiocyanate derivative according to claim 1, wherein X is -O- and R is alkyl, cycloalkyl or alkynyl.

5. A process for producing an aromatic isothiocyanate derivative according to claim 1, wherein the aromatic amine derivative is selected from 4-chloro-2-fluoro-5-hydroxyaniline, 4-chloro-2-fluoro-5-(2-propynyl)oxyaniline, 4-chloro-2-fluoro-5-pentyloxycarbonylmethoxyaniline, 4-chloro-2-fluoro-5-cyclopentyloxycarbonylmethylthioaniline, 6-amino-7-fluoro-4-methyl-2H-1,4-benzoxazin-3(4H)-one and 6-amino-7-fluoro-4-(2-propynyl)-2H- 1,4-benzoxazin-3(4H)-one.

6. A process for producing an aromatic isothiocyanate derivative according to any one of claims 1 to 5, wherein the nitrogen-containing polar solvent is a heterocyclic compound or an amide compound.

7. A process for producing an aromatic isothiocyanate derivative according to claim 6, wherein the nitrogen-containing polar solvent is at least one selected from 1-methylimidazole, pyridine, 5-ethyl-2-methylpyridine, 2-picoline, 3-picoline, 4-picoline, N,N-dimethylformamide and 1-methyl-2-pyrrolidone.

8. A process for producing an aromatic isothiocyanate derivative according to any one of claims 1 to 7, wherein the base is an inorganic base.

9. A process for producing an aromatic isothiocyanate derivative according to any one of claims 1 to 7,
wherein the base is at least one selected from sodium carbonate, potassium carbonate, triethylamine, trimethylamine, 1-methylpiperidine, 1-methylpyrrolidine, 4-methylmorpholine and 4-dimethylaminopyridine.

## Patentansprüche

1. Verfahren zur Herstellung eines aromatischen Isothiocyanatderivats der allgemeinen Formel [I]: 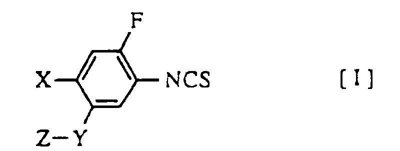 in der X ein Chloratom oder -O- ist; wenn X ein Chloratom ist, steht Y für -O- oder -S- und Z ist ein Wasserstoffatom, ein Alkyl-, Cycloalkyl-, Alkenyl-, Alkinyl-, Alkoxycarbonyl-, Cycloalkoxycarbonyl-, Carboxyalkyl-, Alkoxycarbonylalkyl- oder Cycloalkoxycarbonylalkylrest, oder wenn X für -O- steht, ergeben X und Z zusammen die Gruppe -O-CH₂-CO- und Y steht für -NR-, wobei R ein Wasserstoffatom, ein Alkyl-, Cycloalkyl-, Alkenyl- oder Alkinylrest ist,
wobei das Verfahren die Umsetzung eines aromatischen Aminderivats der allgemeinen Formel [II]: 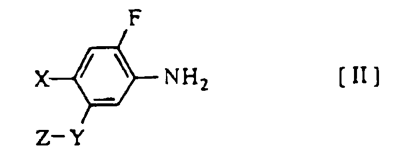 in der X, Y und Z jeweils wie vorstehend definiert sind, mit Kohlenstoffdisulfid in Gegenwart einer Base in einem stickstoffhaltigen polaren Lösungsmittel und anschließend mit einem Alkylchlorcarbonat umfaßt.

2. Verfahren zur Herstellung eines aromatischen Isothiocyanatderivats nach Anspruch 1, wobei X ein Chloratom ist, Y für -O- steht und Z ein Wasserstoffatom, ein Alkinyl-, Alkoxycarbonylalkyl- oder Cycloalkoxycarbonylalkylrest ist.

3. Verfahren zur Herstellung eines aromatischen Isothiocyanatderivats nach Anspruch 1, wobei X ein Chloratom ist, Y für -S- steht und Z ein Alkoxycarbonylalkyl- oder Cycloalkoxycarbonylalkylrest ist.

4. Verfahren zur Herstellung eines aromatischen Isothiocyanatderivats nach Anspruch 1, wobei X für -O- steht und R ein Alkyl-, Cycloalkyl- oder Alkinylrest ist.

5. Verfahren zur Herstellung eines aromatischen Isothiocyanatderivats nach Anspruch 1, wobei das aromatische Aminderivat ausgewählt wird aus 4-Chlor-2-fluor-5-hydroxyanilin, 4-Chlor-2-fluor-5-(2-propinyl)oxyanilin, 4-Chlor-2-fluor-5-pentyloxycarbonylmethoxyanilin, 4-Chlor-2-fluor-5-cyclopentyloxycarbonylmethylthioanilin, 6-Amino-7-fluor-4-methyl-2H-1,4-benzoxazin-3 (4H)-on und 6-Amino-7-fluor-4-(2-propinyl)-2H-1,4-benzoxazin-3(4H)-on.

6. Verfahren zur Herstellung eines aromatischen Isothiocyanatderivats nach einem der Ansprüche 1 bis 5, wobei das stickstoffhaltige polare Lösungsmittel eine heterocyclische Verbindung oder eine Amidverbindung ist.

7. Verfahren zur Herstellung eines aromatischen Isothiocyanatderivats nach Anspruch 6, wobei das stickstoffhaltige polare Lösungsmittel wenigstens eines ist, ausgewählt aus 1-Methylimidazol, Pyridin, 5-Ethyl-2-methylpyridin, 2-Picolin, 3-Picolin, 4-Picolin, N,N-Dimethylformamid und 1-Methyl-2-pyrrolidon.

8. Verfahren zur Herstellung eines aromatischen Isothiocyanatderivats nach einem der Ansprüche 1 bis 7, wobei die Base eine anorganische Base ist.

9. Verfahren zur Herstellung eines aromatischen Isothiocyanatderivats nach einem der Ansprüche 1 bis 7, wobei die Base wenigstens eine ist, ausgewählt aus Natriumcarbonat, Kaliumcarbonat, Triethylamin, Trimethylamin, 1-Methylpiperidin, 1-Methylpyrrolidin, 4-Methylmorpholin und 4-Dimethylaminopyridin.

## Revendications

1. Procédé de production d'un dérivé isothiocyanate aromatique de formule générale [I]: 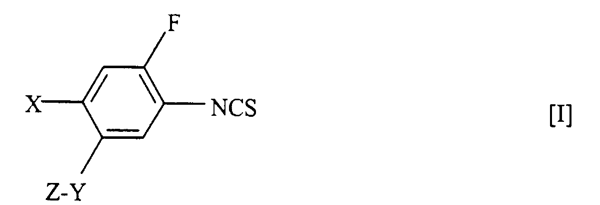 où X est un chlore ou -O- ; quand X est le chlore, Y est -O- ou -S-, et Z est un hydrogène, alkyle, cycloalkyle, alcényle, alcynyle, alcoxycarbonyle, cylcoalcoxycarbonyle, carboxyalkyle, alcoxycarbonylalkyle ou cycloalcoxycarbonylalkyle, ou quand X est -O-, X et Z sont pris ensemble pour former -O-CH₂-CO-, et Y est -NR- où R est un hydrogène, alkyle, cycloalkyle, alcényle ou alcynyle, ce procédé comprend de faire réagir un dérivé amine aromatique de formule générale [II]: 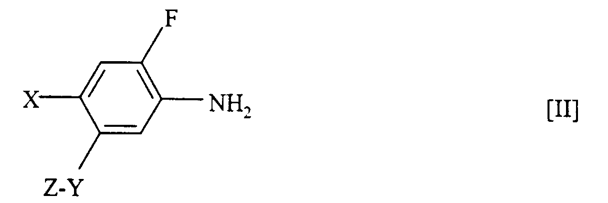 où X, Y et Z sont tels que défini ci-dessus avec le disulfure de carbone en présence d'une base dans un solvant polaire contenant de l'azote, et ensuite avec un chlorocarbonate d'alkyle.

2. Procédé de production d'un dérivé isothiocyanate aromatique selon la revendication 1, où X est le chlore, Y est -O- et Z est un hydrogène, alcynyle, alcoxycarbonylalkyle ou cycloalcoxycarbonylalkyle.

3. Procédé de production d'un dérivé isothiocyanate aromatique selon la revendication 1, où X est le chlore, Y est -S- et Z est un alcoxycarbonylalkyle ou cycloalcoxycarbonylalkyle.

4. Procédé de production d'un dérivé isothiocyanate aromatique selon la revendication 1, où X est -O- et R est un alkyle, cycloalkyle ou alcynyle.

5. Procédé de production d'un dérivé isothiocyanate aromatique selon la revendication 1, où le dérivé amine aromatique est choisi parmi les 4-chloro-2-fluoro-5-hydroxyaniline, 4-chloro-2-fluoro-5-(2-propynyl)oxyaniline, 4-chloro-2-fluoro-5-pentyloxycarbonylméthoxyaniline, 4-chloro-2-fluoro-5-cyclopentyloxycarbonylméthylthioaniline, 6-amino-7-fluoro-4-méthyl-2H-1,4-benzoxazine-3(4H)-one et 6-amino-7-fluoro-4-(2-propynyl)-2H-1,4-benzoxazine-3-(4H)-one.

6. Procédé de production d'un dérivé isothiocyanate aromatique selon l'une quelconque des revendications 1 à 5, où le solvant polaire contenant de l'azote est un composé hétérocyclique ou un composé amide.

7. Procédé de production d'un dérivé isothiocyanate aromatique selon la revendication 6, où le solvant polaire contenant de l'azote est au moins l'un des produits choisis parmi les 1-méthylimidzole, pyridine, 5-éthyl-2-méthylpyridine, 2-picoline, 3-picoline, 4-picoline, N,N-diméthylformamide et 1-méthyl-2-pyrrolidone.

8. Procédé de production d'un dérivé isothiocyanate aromatique selon l'une quelconque des revendications 1 à 7, où la base est une base minérale.

9. Procédé de production d'un dérivé isothiocyanate aromatique selon l'une quelconque des revendications 1 à 7,
où la base est au moins un composé choisi parmi les carbonate de sodium, carbonate de potassium, triéthylamine, thriméthylamine, 1-méthylpipéridine, 1-méthylpyrrolidine, 4-méthylmorpholine et 4-diméthylaminopyridine.
